# EUROPEAN PATENT APPLICATION

(11) **EP 1 136 549 A1**
(43) Date of publication of application: **26.09.2001**
(21) Application number: 99969738.6
(22) Date of filing: 29.09.1999
(51) Int. Cl.: C12N 9/64, C12N 1/21, C12N 15/57, C12P 21/02, C12Q 1/37, A61K 38/57

(54) **DNAS ENCODING NOVEL POLYPEPTIDES**

(30) Priority: 29.09.1998 JP 27625898; 29.09.1998 JP 29150598
(71) Applicant: Seiki, Motoharu, Shinagawa-ku, Tokyo 142-0061 (JP)
(72) Inventor: Seiki, Motoharu, Shinagawa-ku, Tokyo 142-0061 (JP)
(74) Representative: VOSSIUS & PARTNER
(86) International application number: JP9905349
(87) International publication number: WO0018900

(57) **Abstract**

The present invention provides a novel, membrane-type matrix metalloproteinase polypeptide [MT4-MMP(2)] that has, different from the hitherto reported MT4-MMP, physiological activity; DNAs encoding the metalloproteinase polypeptide; a method of producing the metalloproteinase polypeptide; and a method of screening for inhibitors or activators of the metalloproteinase polypeptide using the metalloproteinase polypeptide and the DNAs.

The present invention also provides novel, human and mouse membrane-type matrix metalloproteinase polypeptides (MT5-MMPs) having physiological activity; DNAs encoding the metalloproteinase polypeptides; a method of producing the metalloproteinase polypeptides; and a method of screening for inhibitors or activators of the metalloproteinase polypeptides using the metalloproteinase polypeptides and the DNAs.

## Description

### TECHNICAL FIELD

The present invention relates to novel membrane-type matrix metalloproteinase polypeptides; DNAs encoding the polypeptides; vectors comprising the DNAs; transformants transformed with the vectors; and a method of producing the polypeptides. Furthermore, the present invention relates to a method of searching for inhibitors or activators of the polypeptides using the polypeptides, a part thereof, or microorganisms or animal cells expressing the polypeptides or a part thereof, as well as a method of searching for compounds that regulate the gene expression of the polypeptides.

### BACKGROUND ART

A group of enzymes generically termed "matrix metalloproteinases" (hereinafter, abbreviated to MMPs) that have metal ions at the active center are involved in the degradation of extracellular matrix composed of complicated components such as collagens, fibronectin, laminin and proteoglycans.

To date, the following MMPs have been reported: interstitial collagenase (MMP-1), gelatinase A (MMP-2), gelatinase B (MMP-9), stromelysin 1 (MMP-3), matrilysin (MMP-7), neutrophil collagenase (MMP-8), stromelysin 2 (MMP-10), stromelysin 3 (MMP-11), metallo-elastase (MMP-12), collagenase 3 (MMP-13), membrane type 1 MMP (MT1-MMP or MMP-14), membrane type 2 MMP (MT2-MMP or MMP-15), membrane type 3 MMP (MT3-MMP or MMP-16), membrane type 4 MMP (MT4-MMP or MMP-17), etc. (Protein, Nucleic Acid and Enzyme, 42, 2386 (1997)). These MMPs form a family, and each MMP is basically composed of an N-terminal propeptide domain, an active domain to which zinc ions bind, and a hemopexin-like domain. In MMP-7, no hemopexin-like domain is found. Transmembrane-type MMPs have a transmembrane domain and a intracellular domain at the C-terminal of the hemopexin-like domain.

A human MT4-MMP gene has already been reported [Puente, Cancer Research, 56, 944 (1996)]. However, a translation initiation site is not included in the nucleotide sequence of this gene, and this gene was defined as a human MT4-MMP gene simply because it comprises a nucleotide sequence containing MMP-like domains. Thus, it is difficult to consider that this gene encodes the full-length of MT4-MMP.

On the other hand, it is known that production of MT1-MMP is enhanced in patients with arthrosis deformans [Am. J. Pathol., 151, 245 (1997)]; that MMPs are important for the infiltration of leukocytes into tissues that is important in immunological and inflammatory reactions [J. Immunol., 156, 1 (1996)]; that MMP inhibitors prevent hepatitis [Eur. J. Pharmacol., 341, 105 (1998)]; and that MMP inhibitors are effective for treating corneal ulcer [Japanese Journal of Ophthalmology, 102, 270 (1998)].

It is also known that MMPs are important for cancer proliferation, infiltration and metastasis [Protein, Nucleic Acid and Enzyme, 42, 2386 (1997)], and it is reported that MMP inhibitors have carcinostatic activity [SCRIP, 2349, 20 (1998)].

Furthermore, it is suggested that MT4-MMP is expressed in leukocytes and thus may be involved in the migration and infiltration of leukocytes.

From what have been described so far, MMPs may be used for markers for diagnosing arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury or inflammations associated with infiltration of leukocytes, and inhibitors of MMPs are useful for preventing or treating these diseases.

The already reported MT4-MMP gene [Cancer Research, 56, 944 (1996)] does not include a transcription initiation site nor has such a domain structure as seen in known membrane-type MMPs such as MT1-MMP. Therefore, this gene represents a sequence encoding a non-physiological peptide not expressed *in vivo.*

The present invention provides a novel, membrane-type matrix metalloproteinase polypeptide [hereinafter, sometimes abbreviated to MT4-MMP(2)] that is, different from the hitherto reported MT4-MMP, physiologically active; a DNA encoding the metalloproteinase polypeptide; a method of producing the metalloproteinase polypeptide; and a method of screening for inhibitors or activators of the metalloproteinase polypeptide using the polypeptide or the DNA encoding the polypeptide.

The present invention also provides physiologically active, novel, human and mouse membrane-type matrix metalloproteinase polypeptides (hereinafter, abbreviated to human or mouse MT5-MMP); DNAs encoding the metalloproteinase polypeptides; a method of producing the metalloproteinase polypeptides; and a method of screening for inhibitors or activators of the metalloproteinase polypeptides using the polypeptides or the DNAs encoding the polypeptides.

### DISCLOSURE OF THE INVENTION

The present inventor has made intensive and extensive researches based on the assumption that the known human MT4-MMP is not a protein having the inherent activity of MT4-MMP and that a true MT4-MMP protein having the activity should exist. Thus, the present invention has been achieved.

Also, the present inventor has made intensive and extensive researches based on the assumption that useful and novel membrane-type MMPs should exist other than hitherto known membrane-type MMPs that are considered useful in pharmaceutical purposes. Thus, the present invention has been achieved.

The present invention relates to the following inventions (1) to (32).
(1) A polypeptide consisting of the amino acid sequence as shown in SEQ ID NO: 1.
(2) A polypeptide having an amino acid sequence wherein one or several amino acids are deleted, substituted or added in the amino acid sequence of the polypeptide of (1) above and having metalloproteinase activity.
(3) A polypeptide consisting of the amino acid sequence as shown in SEQ ID NO: 2.
(4) A polypeptide having an amino acid sequence wherein one or several amino acids are deleted, substituted or added in the amino acid sequence of the polypeptide of (3) above and having metalloproteinase activity.
   The deletion, substitution or addition mentioned in (2) and (4) above can be made by site-specific mutagenesis that was a well-known technique prior to the filing of the present application. "One or several amino acids" means the number of amino acids that can be deleted, substituted or added by site-specific mutagenesis. The polypeptide having an amino acid sequence wherein one or several amino acids are deleted, substituted or added in the amino acid sequence and having metalloproteinase activity can be prepared based on those methods described in Molecular Cloning: A Laboratory Manual, Second Edition, Cold Spring Harbor Laboratory Press (1989) (hereinafter, abbreviated to Molecular Cloning 2nd Ed.); Current Protocols in Molecular Biology, Supplement 1-38, John Wiley & Sons (1987-1997) (hereinafter, abbreviated to Current Protocols 1-38); Nucleic Acid Research, 10, 6487 (1982); Proc. Natl. Acad. Sci. USA, 79, 6409 (1982); Gene, 34, 315 (1985), Nucleic Acids Research, 13, 4431 (1985); Proc. Natl. Acad. Sci. USA, 82, 488 (1985); Proc. Natl. Acad. Sci. USA, 81, 5662 (1984); Science, 224, 1431 (1984); PCT WO85/00817 (1985); Nature, 316, 601 (1985) and so forth.
(5) A polypeptide consisting of the amino acid sequence as shown in SEQ ID NO: 5.
(6) A polypeptide having an amino acid sequence wherein one or several amino acids are deleted, substituted or added in the amino acid sequence of the polypeptide of (5) above and having metalloproteinase activity.
(7) A polypeptide consisting of the amino acid sequence as shown in SEQ ID NO: 6.
(8) A polypeptide having an amino acid sequence wherein one or several amino acids are deleted, substituted or added in the amino acid sequence of the polypeptide of (7) above and having metalloproteinase activity.
(9) A DNA encoding the polypeptide of any one of (1) to (4) above.
(10) A DNA encoding the polypeptide of any one of (5) to (8) above.
(11) A DNA consisting of the nucleotide sequence of positions 86-1846 of SEQ ID NO: 3 or positions 100-1917 of SEQ ID NO: 4, or a DNA which hybridizes to the DNA under stringent conditions and which encodes a polypeptide having metalloproteinase activity.
   The above expression "a DNA which hybridizes under stringent conditions" means a DNA that is obtained by colony hybridization, plaque hybridization, Southern blot hybridization or the like using, as a probe, a DNA consisting of the nucleotide sequence of positions 86-1846 of SEQ ID NO: 3 or positions 100-1917 of SEQ ID NO: 4. Specifically, a DNA which can be identified by carrying out a hybridization at 65°C in the presence of 0.7-1.0 mol/L NaCI using a filter on which the DNA derived from colony or plaque is immobilized, and then washing the filter in 0.1-2 x SSC (saline-sodium citrate) solution (1x SSC solution is composed of 150 mmol/L sodium chloride and 15 mmol/L sodium citrate) at 65°C.
   Hybridization may be carried out based on those methods described in laboratory manuals such as Molecular Cloning 2nd Ed., Current Protocol in Molecular Biology, and DNA Cloning 1: Core Techniques, A Practical Approach, Second Edition, Oxford University Press (1995).
   Specific examples of hybridizable DNAs include DNAs having at least 80% or more, preferably 95% or more homology to the nucleotide sequence of positions 86-1846 of SEQ ID NO: 3 or positions 100-1917 of SEQ ID NO: 4.
(12) A DNA consisting of the nucleotide sequence of positions 75-1928 of SEQ ID NO: 7 or positions 1-1935 of SEQ ID NO: 8, or a DNA which hybridizes to the DNA under stringent conditions and which encodes a polypeptide having metalloproteinase activity.
   The above expression "a DNA which hybridizes under stringent conditions" means a DNA that is obtained by colony hybridization, plaque hybridization, Southern blot hybridization or the like using, as a probe, a DNA consisting of the nucleotide sequence of positions 75-1928 of SEQ ID NO: 7 or positions 1-1935 of SEQ ID NO: 8. Specifically, a DNA which can be identified by carrying out a hybridization at 65°C in the presence of 0.7-1.0 mol/L NaCl using a filter on which the DNA derived from colony or plaque is immobilized, and then washing the filter in 0.1-2 x SSC (saline-sodium citrate) solution (1x SSC solution is composed of 150 mmol/L sodium chloride and 15 mmol/L sodium citrate) at 65°C.
   Specific examples of hybridizable DNAs include DNAs having at least 80% or more, preferably 95% or more homology to the nucleotide sequence of positions 75-1928 of SEQ ID NO: 7 or positions 1-1935 of SEQ ID NO: 8.
(13) A recombinant DNA that is obtained by integrating the DNA of any one of (9) to (12) above into a vector.
(14) A transformant comprising the recombinant DNA of (13) above.
(15) The transformant of (14) above, wherein said transformant is a microorganism belonging to the genus *Escherichia.*
(16) The transformant of (15) above, wherein said microorganism belonging to the genus *Escherichia is Escherichia coli*.
(17) A method of producing the polypeptide of any one of (1) to (8) above, comprising culturing a transformant comprising a recombinant DNA obtained by integrating a DNA encoding the polypeptide into a vector in a medium, allowing the polypeptide to be produced and accumulated in the culture, and recovering the polypeptide from the culture.
(18) An oligonucleotide selected from an oligonucleotide having a nucleotide sequence identical with a nucleotide sequence consisting of consecutive 5 to 60 bases of the DNA of (9) or (11) above; an oligonucleotide having a nucleotide sequence complementary to the nucleotide sequence of said oligonucleotide; or an oligonucleotide derivative of any one of the above oligonucleotides.
(19) An oligonucleotide selected from an oligonucleotide having a nucleotide sequence identical with a nucleotide sequence consisting of consecutive 5 to 60 bases of the DNA of (10) or (12) above; an oligonucleotide having a nucleotide sequence complementary to the nucleotide sequence of said oligonucleotide; or an oligonucleotide derivative of any one of the above oligonucleotides.
(20) A method of detecting an mRNA encoding the polypeptide of any one of (1) to (8) above using the oligonucleotide of (18) or (19) above.
(21) A method of inhibiting expression of the polypeptide of any one of (1) to (8) using the oligonucleotide of (18) or (19) above.
(22) A method of screening for an inhibitor or an activator of the polypeptide of any one of (1) to (8) above, which comprises using the polypeptide and a cell that expresses the polypeptide.
(23) A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury or inflammations associated with infiltration of leukocytes, wherein said agent comprises the polypeptide of any one of (1) to (4) above.
(24) A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury, inflammations associated with infiltration of leukocytes, brain disorders at the time of cerebral apoplexy, Alzheimer's disease, dementia, multiple sclerosis, Parkinson's disease or brain tumor, wherein said agent comprises the polypeptide of any one of (5) to (8) above.
(25) A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury or inflammations associated with infiltration of leukocytes, wherein said agent comprises the DNA of (9) or (11) above.
(26) A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury, inflammations associated with infiltration of leukocytes, brain disorders at the time of cerebral apoplexy, Alzheimer's disease, dementia, multiple sclerosis, Parkinson's disease or brain tumor, wherein said agent comprises the DNA of (10) or (12) above.
(27) A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury or inflammations associated with infiltration of leukocytes, wherein said agent comprises the oligonucleotide of (18) above.
(28) A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury, inflammations associated with infiltration of leukocytes, brain disorders at the time of cerebral apoplexy, Alzheimer's disease, dementia, multiple sclerosis, Parkinson's disease or brain tumor, wherein said agent comprises the oligonucleotide of (19) above.
(29) A vector for gene therapy for treating arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury or inflammations associated with infiltration of leukocytes, wherein said vector is obtained by integrating the DNA of (9) or (11) above, or the oligonucleotide of (18) above into a vector.
(30) A vector for gene therapy for treating arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury, inflammations associated with infiltration of leukocytes, brain disorders at the time of cerebral apoplexy, Alzheimer's disease, dementia, multiple sclerosis, Parkinson's disease or brain tumor, wherein said vector is obtained by integrating the DNA of (10) or (12) above, or the oligonucleotide of (19) above into a vector.
(31) A method of screening for a compound that regulates the expression of a gene encoding the polypeptide of any one of (1) to (8) above, which comprises contacting a cell that expresses the polypeptide with a test sample.
(32) The method of (31) above, wherein said compound that regulates the expression of a gene is detected by determining the amount of mRNA encoding the polypeptide of any one of (1) to (8) above.

### BRIEF DESCRIPTION OF THE DRAWINGS

Fig. 1 shows an alignment of the amino acid sequences of human MT5-MMP and mouse MT5-MMP with the amino acid sequences of human MT1-MMP, MT2-MMP, MT3-MMP and MT4-MMP(2).

Mark "*" indicates identical amino acid residues.

Mark "." indicates similar amino acid residues.

(Amino acid residues are represented by one-letter abbreviations.)

In this Figure, "kb" means kilobase pairs.

Fig. 2 shows the results of an experiment in which a mouse MT5-MMP partial peptide (i.e., propeptide domain + active domain; "MT5ΔC" in this Figure) of various concentrations was reacted with pro-MMP-2 to thereby examine the peptide's ability to cleave and activate pro-MMP-2.

As a positive control, APMA was used. As a result, activation was recognized in an MMP concentration dependent manner. In this Figure, "Active" shows activated MMP-2.

### BEST MODES FOR CARRYING OUT THE INVENTION

Hereinbelow, the present invention will be described in detail.

### [1] Acquisition of the DNAs encoding the Novel Matrix Metalloproteinase Polypeptides of the Present Invention

### (1) Preparation of cDNA Libraries

In order to construct a cDNA library, total RNA or mRNA is prepared from an appropriate cell or tissue.

As a method for preparing total RNA, the guanidine thiocyanate-cesium trifluoroacetate method [Methods in Enzymology, 154, 3 (1987)], the acid guanidine thiocyanate/phenol/chloroform (AGPC) method [Analytical Biochemistry, 162, 156 (1987); Experimental Medicine, 9, 1937 (1991)]; or the like may be used.

As a method for preparing mRNA (as poly(A)⁺ RNA) from total RNA, a method using oligo(dT) immobilized cellulose column (Molecular Cloning 2nd Ed.), a method using oligo(dT) latex [Cell Engineering, Supplement 8, "New Cell Engineering Experiment Protocols", SHUJUNSHA Co., pp.48-52; Nucleic Acids Res., Symposium Series, 19, 61 (1988)] or the like may be used.

Alternatively, mRNA may be prepared directly from a tissue or cell using a commercial kit such as First Track mRNA Isolation Kit (Invitrogen) or Quick Prep mRNA Purification Kit (Pharmacia).

In the case of MT4-MMP(2), preferably, types of cDNA libraries which contained ESTs of the DNA encoding MT4-MMP found in databases are ascertained, and then cells or tissues that were used for the construction of those libraries, or cell strains or the like derived from those tissues may be used as an appropriate cell or tissue. In the case of MT5-MMP, it is preferable to use tissues such as brain and kidney or cell strains derived from those tissues as an appropriate cell or tissue.

From the resultant total RNA or mRNA, a cDNA library is constructed by conventional methods.

Specific examples of methods for constructing cDNA libraries include those described in Molecular Cloning 2nd Ed.; Current Protocols 1-38; DNA Cloning 1: Core Techniques, A practical Approach, Second Edition, Oxford University Press (1995); etc. or methods using commercial kits such as Superscript Plasmid System for cDNA Synthesis and Plasmid Cloning manufactured by Gibco BRL and ZAP-cDNA Synthesis Kit manufactured by Stratagene.

As a cloning vector for constructing a cDNA library, any vector, such as a phage vector or plasmid vector, may be used as long as it is capable of autonomous replication in E. *coli* K12 strain.

Specifically, ZAP Express [Stratagene; Strategies, 5, 58 (1992)], pBluescript II SK(+) [Nucleic Acids Research, 17, 9494 (1989)], Lamda ZAP II {Stratagene), λ gt10, λgt11 [DNA Cloning, A Practical Approach, 1, 49 (1985)], λ TriplEx (Clontech), λ ExCell (Pharmacia), pT7T318U (Pharmacia), pcD2 [Mol. Cell. Biol., 3, 280 (1983)], pUC18 [Gene, 33, 103 (1985)], pAMo [J. Biol. Chem., 268, 22782-22787 (1993); also called as "pAMoPRC3Sc" (Japanese Unexamined Patent Publication No. 05-336963) or the like may be used.

As a host microorganism, any microorganism may be used as long as it belongs to *Escherichia coli*. Specifically, *Escherichia coli* XL1-Blue MRF' [Stratagene; Strategies 5, 81 (1992)], *Escherichia coli* C600 [Genetics, 39, 440 (1954)], *Escherichia coli* Y1088 [Science, 222, 778 (1983)], *Escherichia coli* Y1090 [Science, 222, 778 (1983)], *Escherichia coli* NM522 [J. Mol. Biol., 166, 1 (1983)], *Escherichia coli* K802 [J. Mol. Biol., 16, 118 (1966)], *Escherichia coli* JM105 [Gene, 38, 275 (1985)], *Escherichia coli* SOLRTM Strain (Stratagene), *Escherichia coli* LE392 (Molecular Cloning 2nd Ed.) or the like may be used.

In addition to cDNA libraries constructed by the above-described methods, commercial cDNA libraries may also be used.

Examples of commercial cDNA libraries include cDNA libraries of individual organs derived from animals such as human, bovine, mouse, rat or rabbit manufactured by Clontech, Lifetech Oriental, etc.

### (2) Aquisition of the DNAs of the Invention

cDNA clones containing the DNA of the present invention may be selected from the cDNA library prepared in (1) above by such method as colony hybridization or plaque hybridization (Molecular Cloning 2nd Ed.) using a radioactively or fluorescently labeled probe.

As a probe for MT4-MMP(2) gene, an oligonucleotide based on the nucleotide sequence of a DNA encoding MT4-MMP (a part of which has been elucidated) may be used. For MT5-MMP gene, an oligonucleotide based on the nucleotide sequence of a DNA encoding MT3-MMP may be used.

From the resultant clones of interest, mRNA is obtained as described above and then cDNA is synthesized.

An adaptor is added to both ends of the resultant cDNA. Using a primer based on the sequence of this adaptor and a gene-specific primer based on the partially known sequence of the gene of interest, 5' RACE (rapid amplification of cDNA ends) and 3' RACE [Proc. Natl. Acad. Sci. USA, 85, 8998 (1988)] are carried out to obtain a cDNA fragment located 5' to the primer sequence and a cDNA fragment located 3' to the primer sequence.

By ligating the resultant cDNA fragments, a full-length cDNA can be obtained.

The nucleotide sequence of the thus obtained DNA fragment can be determined by integrating into a vector the fragment as it is or the fragment digested with an appropriate restriction enzyme by conventional methods and then analyzing the sequence by conventional methods such as the dideoxy method by Sanger et al. [Proc. Natl. Acad. Sci. USA, 74, 5463 (1977)] or with a DNA sequencer manufactured by Perkin Elmer (373A DNA Sequencer), Pharmacia, LI-COR, etc.

In order to determine the nucleotide sequence of the genomic DNA fragment encoding the polypeptide of the present invention, conventional methods for chromosomal DNA cloning (Molecular Cloning 2nd Ed.) can be used.

Briefly, chromosomal DNA from cells expressing the polypeptide of the present invention [such as monocytic THP-1 cells for MT4-MMP(2); such as brain or kidney cells for MT5-MMP] is digested with a restriction enzyme. The digested fragments are cloned into a conventional plasmid vector or phage vector to construct a genomic library.

The genomic library is screened using, as a probe, the DNA fragment obtained and sequenced as described above in the same manner as in the cDNA cloning described above. Thus, clones containing the genomic gene encoding the polypeptide of the present invention can be obtained.

Using the resultant clones, the nucleotide sequence of the genomic gene can be determined by the above-described method.

It is also possible to obtain a DNA of interest derived from other tissues or other animals (e.g. human) by selecting DNAs that hybridize to the DNA obtained by the above-described method under stringent conditions.

Alternatively, a DNA of interest may be chemically synthesized with a DNA synthesizer based on the nucleotide sequence information obtained by the above-described method. As a DNA synthesizer, one using the thiophosphite method manufactured by Shimadzu Corp., a DNA synthesizer model 392 using the phosphoamidite method manufactured by Perkin Elmer, or the like may be enumerated.

The novelty of the nucleotide sequence obtained can be confirmed by searching DNA sequence databases of GenBank, EMBL, DDBJ, etc. using a homology search program such as BLAST. If the nucleotide sequence is found to be novel, it is converted into an amino acid sequence. Then, amino acid sequence databases of GenPept, PIR, Swiss-Prot, etc. are searched using a homology search program such as FASTA or FrameSearch to thereby search for existing genes having homology to the novel nucleotide sequence.

As a DNA encoding MT4-MMP(2), the polypeptide of the present invention, that has been confirmed to have a novel nucleotide sequence by the above-described method, a DNA having the nucleotide sequence as shown in SEQ ID NO: 3 or SEQ ID NO: 4 may be given, for example.

As a plasmid comprising a DNA having the nucleotide sequence as shown in SEQ ID NO: 3, plasmid pmMT4/pBSSK may be given. As a plasmid comprising a DNA having the nucleotide sequence as shown in SEQ ID NO: 4, plasmid phMT4/pBSIIKS may be given.

*Escherichia coli* pmMT4/pBSSK comprising plasmid pmMT4/pBSSK and *Escherichia coli* phMT4/pBSIIKS comprising plasmid phMT4/pBSIIKS were deposited as FERM BP-6528 and FERM BP-6530, respectively, on September 25, 1998 with National Institute of Bioscience and Human-Technology, Agency of Industrial Science and Technology located at 1-3, Higashi 1-chome, Tsukuba City, Ibaraki Pref., Japan (postal code: 305-8566).

As a DNA encoding MT5-MMP, another polypeptide'of the present invention, that has been confirmed to have a novel nucleotide sequence by the above-described method, a DNA having the nucleotide sequence as shown in SEQ ID NO: 7 or SEQ ID NO: 8 may be given, for example.

As a plasmid comprising a DNA having the nucleotide sequence as shown in SEQ ID NO: 7, plasmid pmMT5/pBSSK may be given. As a plasmid comprising a DNA having the nucleotide sequence as shown in SEQ ID NO: 8, plasmid phMT5/pGEM may be given.

*Escherichia coli* pmMT5/pBSSK comprising plasmid pmMT5/pBSSK and *Escherichia coli* phMT5/pGEM comprising plasmid phMT5/pGEM were deposited as FERM BP-6529 and FERM BP-6531, respectively, on September 25, 1998 with National Institute of Bioscience and Human-Technology, Agency of Industrial Science and Technology located at 1-3, Higashi 1-chome, Tsukuba City, Ibaraki Pref., Japan (postal code: 305-8566).

### (3) Preparation of the Oligonucleotides of the Invention

Using the DNAs and DNA fragments of the present invention obtained in the above-described method, oligonucleotides (anti-sense and sense) comprising a partial sequence of the DNAs of the present invention can be prepared by conventional methods or with the DNA synthesizer mentioned above.

Examples of such oligonucleotides include a DNA having the same sequence as that of consecutive 5 to 60 bases within the nucleotide sequences of the above-described DNAs, or a DNA complementary thereto. Specifically, as oligonucleotides comprising a partial sequence of MT4-MMP(2) gene, a DNA having a nucleotide sequence identical with a nucleotide sequence consisting of consecutive 5 to 60 bases in the nucleotide sequence as shown in SEQ ID NO: 3 or 4, or a DNA complementary thereto may be given. As oligonucleotides comprising a partial sequence of MT5-MMP gene, a DNA having a nucleotide sequence identical with a nucleotide sequence consisting of consecutive 5 to 60 bases in the nucleotide sequence as shown in SEQ ID NO: 7 or 8, or a DNA complementary thereto may be given. When oligonucleotides are used as a sense primer and an anti-sense primer, the above-described oligonucleotides are preferably used which are not greatly different between the selected two in melting temperature (Tm) and in the number of bases.

Furthermore, derivatives of these oligonucleotides may also be used as the oligonucleotide of the present invention.

Some of the examples of such oligonucleotide derivatives include oligonucleotide derivatives in which phosphodiester bonds are converted to phosphorothioate bonds; oligonucleotide derivatives in which phosphodiester bonds are converted to N 3'-P5' phosphoamidate bonds; oligonucleotide derivatives in which ribose and phosphodiester bonds are converted to peptide-nucleic acid bonds; oligonucleotide derivatives in which uracil is substituted by C-5 propynyluracil; oligonucleotide derivatives in which uracil is substituted by C-5 thiazoluracil; oligonucleotide derivatives in which cytosine is substituted by C-5 propynylcytosine; oligonucleotide derivatives in which cytosine is substituted by phenoxazine-modified cytosine; oligonucleotide derivatives in which ribose is substituted by 2'-O-propylribose; or oligonucleotide derivatives in which ribose is substituted by 2'-methoxyethoxyribose [Cell Engineering, 16, 1463 (1997)].

### [2] Preparation of the Matrix Metalloproteinase Polypeptides of the Invention

### (1) Preparation of Transformants

In order to express in a host cell the DNA of the present invention obtained by the method described in [1] above, methods described in Molecular Cloning 2nd Ed. and Current Protocols 1-38, for example, may be used.

Briefly, a recombinant expression vector is prepared by inserting the DNA of the present invention downstream of a promoter in an appropriate vector. Then, by introducing the recombinant vector into a host cell, a transformant that expresses the polypeptide of the present invention can be obtained.

As a host cell, any cell such as a bacterium, yeast, animal cell, or insect cell may be used as long as it is capable of expressing the gene of interest.

As an expression vector, a vector which is capable of autonomously replicating or integrating into chromosome in the above host cell, and which comprises a promoter at a site appropriate for transcription of the DNA of the present invention, is used.

When a procaryote such as a bacterium is used as the host cell, it is preferred that the expression vector for the polypeptide gene of the present invention be capable of autonomous replication in the procaryote and, at the same time, a recombinant vector composed of a promoter, a ribosome binding sequence, the DNA of the present invention, and a transcription termination sequence. The vector may also contain a gene that controls the promoter.

Examples of expression vectors which may be used in the present invention include pKK233-2 (Pharmacia), pSE280 (Invitrogen), pGEMEX-1 (Promega), pQE-8 (Qiagen), pKYP10 (Japanese Unexamined Patent Publication No. 58-110600), pKYP200 [Agric. Biol. Chem., 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (Stratagene), pGEX (Pharmacia), and pET-3 (Novagen).

As a promoter, any promoter may be used as long as it can direct the expression of the gene of interest in a host cell such as E. *coli* or *Bacillus subtilis*. For example, an *E*. *coli-* or phage-derived promoter such as trp promoter (Ptrp), lac promoter, PL promoter, PR promoter or T7 promoter; SP01 promoter; SP02 promoter; or penP promoter may be used. An artificially designed and altered promoter such as a promoter in which two Prtp promoters are connected in series (Ptrp x 2), tac promoter, lacT7 promoter, or let 1 promoter may also be used.

As a ribosome binding sequence, it is preferable to use a plasmid in which the distance between Shine-Dalgarno sequence and the initiation codon is appropriately adjusted (e.g., 6-18 bp).

In the recombinant vector of the present invention, it is not necessarily required for the expression of the DNA of the present invention to contain a transcription termination sequence, but it is desirable to locate such a sequence immediately downstream of the structural gene.

As a host cell, a microorganism belonging to the genus *Escherichia*, *Serratia*, *Bacillus, Brevibacterium*, *Corynebacterium*, *Microbacterium*, *Pseudomonas* or the like may be used. Specific examples of host cells which may be used in the present invention include *Escherichia coli* XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No.49, *Escherichia coli* W3110, *Escherichia coli* NY49, *Serratiaficaria*, *Serratia fonticola*, *Serratia liquefaciens*, *Serratia marcescens*, *Bacillus subtilis*, *Bacillus amyloliquefaciens*, *Brevibacterium ammmoniagenes*, *Brevibacterium immariophilum* ATCC 14068, *Brevibacterium saccharolyticum* ATCC14066, *Corynebacterium glutamicum* ATCC13032, *Corynebacterium glutamicum* ATCC14067, *Corynebacterium glutamicum* ATCC13869, *Corynebacterium acetoacidophilum* ATCC13870, *Microbacterium ammoniaphilum* ATCC15354, and *Pseudomonas* sp. D-0110.

As a method for introducing the recombinant vector, any method of introducing DNA into the above host cell may be used. For example, the method using calcium ions [Proc. Natl. Acad. Sci., USA, 69, 2110 (1972)], the protoplast method (Japanese Unexamined Patent Publication No. 63-248394), or electroporation [Gene, 17, 107 (1982); Molecular & General Genetics, 168, 111 (1979)] may be used.

When a yeast strain is used as the host cell, an expression vector such as YEp13 (ATCC37115), YEp24 (ATCC37051), YCp50 (ATCC37419), pHS19, or pHS15 may be used.

As a promoter, any promoter that can direct the expression of the gene of interest in yeast may be used. For example, PH05 promoter, PGK promoter, GAP promoter, ADH promoter, gal 1 promoter, gal 10 promoter, heat shock polypeptide promoter, MF α 1 promoter, or CUP 1 promoter may be used.

As a host cell, a yeast strain belonging to the genus *Saccharomyces, Schizosaccharomyces*, *Kluyveromyces*, *Trichosporon*, *Schwanniomyces*, *Pichia* or the like may be used. Specific examples of yest strains that may be used in the present invention include *Saccharomyces cerevisiae*, *Schizosaccharomyces pombe*, *Kluyveromyces lactis*, *Trichosporon pullulans*, *Schwanniomyces alluvius*, *and Pichia pastoris.*

As a method for introducing the recombinant vector, any method of introducing DNA into yeast may be used. For example, electroporation [Methods in Enzymology, 194, 182 (1990)], the spheroplast method [Proc. Nad. Acad. Sci., USA, 81, 4889 (1984)], the lithium acetate method [Journal of Bacteriology, 153, 163 (1983)] or the like may be enumerated.

When an animal cell is used as the host, an expression vector such as pAGE107 (Japanese Unexamined Patent Publication No. 3-22979; Cytotechnology, 3, 133 (1990)], pAS3-3 (Japanese Unexamined Patent Publication No. 2-227075), pCDM8 [Nature, 329, 840 (1987)], pcDNAI/Amp (Invitrogen), pREP4 (Invitrogen), or pAGE103 [Journal of Biochemistry, 101, 1307 (1987)] may be used.

As a promoter, any promoter that can direct the expression of the gene of interest in animal cells may be used. Examples of promoters that may be used in the present invention include the promoter of the IE (immediate early) gene of cytomegalovirus (CMV), the early promoter of SV40, a metallothionein promoter, a retrovirus promoter, a heat shock promoter and SR α promoter. Alternatively, the enhancer of the IE gene of human CMV may be used in combination with the promoter thereof.

Examples of animal cells that may be used in the present invention include human cells such as Namalwa cells, HEK293 cells (ATCC: CRL-1573); simian cells such as COS cells; and Chinese hamster cells such as CHO cells, HBT5637 (Japanese Unexamined Patent Publication No. 63-299).

As a method for introducing the recombinant vector, any method of introducing DNA into animal cells may be used. For example, electroporation [Cytotechnology, 3, 133 (1990)], the calcium phosphate method (Japanese Unexamined Patent Publication No. 2-227075), or lipofection [Proc. Natl. Acad. Sci. USA, 84, 7413 (1987); Virology, 52, 456 (1973)] may be used.

When an insect cell is used as the host, it is possible to express the polypeptide of the present invention according to methods described in, for example, Baculovirus Expression Vectors, A Laboratory Manual, W.H. Freeman and Company, New York (1992); Current Protocols 1-38; and Bio Technology, 6, 47 (1988).

Briefly, a recombinant gene transfer vector and Baculovirus are co-introduced into an insect cell to thereby obtain a recombinant virus in the supernatant of the insect cell culture. Then, the insect cell is infected with the recombinant virus further to allow the production of the polypeptide of the present invention.

As a gene transfer vector that may be used in the above method, pVL1392, pVL1393, pBlueBacIII (all of which are manufactured by Invitrogen) may be enumerated, for example.

As a Baculovirus that may be used in the above method, Autographa californica nuclear polyhedrosis virus that infects insects belonging to the subfamily Hadeniae may be given, for example.

As an insect cell that may be used in the above method, *Spodoptera frugiperda* ovary cells Sf9 and Sf21 [Baculovirus Expression Vectors, A Laboratory Manual (1992)]; a *Trichoplusia ni* ovary cell High5 (Invitrogen); or the like may be enumerated.

As a method of co-introducing a gene transfer vector and Baculovirus into an insect cell for preparing a recombinant virus, the calcium phosphate method (Japanese Unexamined Patent Publication No. 2-227075) or lipofection [Proc. Natl. Acad. Sci. USA, 84, 7413 (1987)] may be enumerated, for example.

As a method of expressing the gene, in addition to direct expression, such as secretion production or fusion protein expression may be carried out based on the methods described in Molecular Cloning 2nd Ed.

When the polypeptide of the present invention is expressed by a yeast strain, animal cell or insect cell, a polypeptide to which sugars or sugar chains have been attached can be obtained.

The polypeptide of the present invention can be prepared by culturing the transformant obtained as described above in a medium, allowing the polypeptide of the present invention to be produced and accumulated in the culture, and recovering the polypeptide from the culture.

It is also possible to express the polypeptide of the present invention in a patient *in vivo* by introducing an appropriate expression vector that directs expression of the polypeptide of the present invention into cells taken from the patient's living body and then returning the cells into the body.

### (2) Culturing of Transformants

The culturing of the transformant of the present invention in a medium is carried out by conventional methods used for culturing hosts.

As a medium to culture the transformant obtained from a procaryotic host such as E. *coli* or an eucaryotic host such as yeast, either a natural or synthetic medium may be used as long as it contains carbon sources, nitrogen sources and inorganic salts assimilable by the microorganism and is suitable for efficient culturing of the transformant.

As carbon sources, any carbon source may be used as long as it is assimilable by the microorganism. For example, carbohydrates such as glucose, fructose, sucrose, or molasses, starch or starch hydrolysate containing them; organic acids such as acetic acid, propionic acid; and alcohols such as ethanol and propanol may be used.

As nitrogen sources, ammonia; ammonium salts of inorganic or organic acids such as ammonium chloride, ammonium sulfate, ammonium acetate, ammonium phosphate; other nitrogen-containing compounds; Peptone; meat extract; yeast extract; corn steep liquor; casein hydrolysate; soybean meal and soybean meal hydrolysate; various fermented microorganism cells and digested products thereof; and the like may be used.

As inorganic substances, potassium dihydrogen phosphate, dipotassium hydrogen phosphate, magnesium phosphate, magnesium sulfate, sodium chloride, iron(II) sulfate, manganese sulfate, copper sulfate, calcium carbonate and the like may be used.

Usually, culturing is carried out under aerobic conditions, by such as shaking culture or submerged aeration agitation culture. The culturing temperature is preferably between 15 to 40°C, and the culturing period is usually 16 to 96 hrs. During the culturing, the pH is maintained at 3.0 to 9.0. The pH adjustment is carried out using an inorganic or organic salt, an alkali solution, urea, calcium carbonate, ammonia or the like.

During the culturing, an antibiotic such as ampicillin or tetracycline may be added to the medium if necessary.

When a microorganism transformed with an expression vector using an inducible promoter is cultured, an inducer may be added to the medium if necessary. For example, when a microorganism transformed with an expression vector using Lac promoter is cultured, isopropyl-*β*-D-thiogalactopyranoside or the like may be added. When a microorganism transformed with an expression vector using trp promoter is cultured, indoleacrylic acid or the like may be added.

As a medium to culture a transformant obtained from an animal cell as a host, commonly used RPMI1640 medium [The Journal of the American Medical Association, 199, 519 (1967)], Eagle's MEM medium [Science, 122, 501 (1952)], DMEM medium [Virology, 8, 396 (1959)], 199 medium [Proceeding of the Society for the Biological Medicine, 73, 1 (1950)] or one of these media supplemented with fetal bovine serum, etc. may be used.

Usually, the culturing is carried out at pH 6-8, at 30-40°C in the presence of 5% CO₂ for 1 to 7 days.

During the culturing, an antibiotic such as kanamycin, penicillin, or streptomycin may be added to the medium if necessary.

As a medium to culture a transformant obtained from an insect cell as a host, commonly used TNM-FH medium (Pharmingen), Sf-900 II SFM medium (Life Technologies), ExCell400 or ExCell405 (both from JRH Biosciences), Grace's Insect Medium [Nature, 195, 788 (1962)] or the like may be used.

Usually, culturing is carried out at pH 6-7 at 25-30°C for 1 to 5 days.

During the culturing, an antibiotic such as gentamycin may be added to the medium if necessary.

### (3) Isolation and Purification of the Expressed Polypeptides

Conventional methods of enzyme isolation/purification may be used to isolate and purify the polypeptides expressed by the method described above from the culture of the above-described transformant. For example, when the polypeptide of the present invention is expressed in a dissolved state in cells, the cells are harvested by centrifugation after completion of the culturing, and then suspended in an aqueous buffer. Subsequently, the cells are disrupted with a sonicator, French press, Manton-Gaulin homogenizer, Dynomill or the like to thereby obtain a cell-free extract, which is then centrifuged to obtain a supernatant. From this supernatant, a purified sample may be obtained by conventional enzyme isolation/purification methods. For example, the solvent extraction method; salting out with ammonium sulfate or the like; desalting; precipitation with organic solvents; anion exchange chromatography using resins such as Q-Sepharose, diethylaminoethyl (DEAE)-Sepharose, DIAION HPA-75 (Mitsubishi Chemical Corp.); cation exchange chromatography using resins such as S-Sepharose FF (Pharmacia); hydrophobic chromatography using resins such as butyl Sepharose, phenyl Sepharose; gel filtration using molecular sieve; affinity chromatography; and electrophoresis such as chromatofocusing, isoelectric focusing; may be used independently or in combination.

When the polypeptide of the present invention is expressed in an insoluble form within cells, the cells are harvested and disrupted in the same manner as described above. Then, the cells are centrifuged to obtain the precipitate fraction, from which the polypeptide is recovered by conventional methods. Subsequently, the polypeptide in an insoluble form is solubilized with a protein-denaturing agent. The resultant solubilized solution is diluted until the solution no longer contains the denaturing agent or the concentration of the denaturing agent becomes so low that no protein denaturation would occur; or the solubilized solution is dialyzed. Thus, the normal steric structure of the polypeptide is restored. Subsequently, a purified sample can be obtained by using the isolation/purification methods described above.

When the polypeptide of the present invention or a derivative thereof (such as sugar-modified polypeptide) is secreted out of cells, the polypeptide or the derivative can be recovered from the culture supernatant. Briefly, the culture is treated by centrifugation, etc. in the same manner as described above to obtain the soluble fraction. From this soluble fraction, a purified sample can be obtained by using the isolation/purification methods described above. When the polypeptide of the present invention or a derivative thereof (such as sugar-modified polypeptide) is expressed on cell surfaces, the membrane fraction of the cultured cells is dissolved with a surfactant to obtain the soluble fraction. From this soluble fraction, a purified sample can be obtained by using the isolation/purification methods described above.

Alternatively, the polypeptide of the present invention may be prepared by chemical synthesis methods such as the Fmoc (fluorenylmethyloxycarbonyl) method and the tBoc (t-butyloxycarbonyl) method. The polypeptide of the present invention may also be chemically synthesized with peptide synthesizers manufactured by Advanced ChemTech, Perkin Elmer, Pharmacia, Protein Technology Instrument, Synthecell-Vega, PerSeptive, Shimadzu Corp. and so forth.

### [3] Detection of the Biological Activity of the Polypeptides of the Invention

The proteinase activity of the polypeptides of the present invention obtained by the method described in [2] above is determined by subjecting a peptide or protein degraded by the polypeptides of the present invention electrophoresis or column chromatography. Alternatively, the activity is determined by measuring degradation of a fluorescence- or isotope-labeled peptide or protein by the polypeptide of the present invention. It is also possible to detect the activity by measuring the state of activation of an enzyme that is activated by excision of a peptide. The activity may also be measured by using a gel containing a peptide that is degraded by the enzyme in the same manner as in gelatin zymography.

### [4] Search for and Identification of Inhibitors or Activators of the Polypeptides of the Invention

A test sample is added to those cells expressing the polypeptide of the present invention prepared by the method described in [2] above, or the polypeptide of the present invention purified by the method described in [2] above from recombinant *E. coli* cells expressing the polypeptide of the present invention prepared by the method described in [2] above.

Then, by comparing the proteinase activity of the polypeptide of the present invention in the presence of the test sample with the activity in the absence of the test sample, it is possible to screen for a substance that enhances the proteinase activity (activator) or a substance that inhibits the proteinase activity (inhibitor).

Specific examples of test samples include synthetic compounds, naturally occurring proteins, artificially synthesized proteins, peptides, saccharides, lipids, modified products or derivatives of these substances; urine, body fluids, tissue extracts, cell culture supematants and cell extracts from mammals (such as mouse, rat, guinea pig, hamster, pig, sheep, bovine, equine, canine, feline, simian, or human); non-peptidic compounds; fermentation products; and extracts from plants and other organisms.

When a peptide is used as a test sample, a random peptide library may be utilized. Examples of random peptide libraries that may be used in the present invention include peptides on phage [Proc. Natl. Acad. Sci. USA, 87, 6378 (1990); PCT Patent Application Number 96/40189] and peptides on plasmids [United States Patent No. 5,270,170; United States Patent No. 5,338,665].

Peptides that bind to MT4-MMP(2) of the present invention can be obtained by screening a random peptide library. Examples of random peptide libraries that may be used in the present invention include peptides on phage [Proc. Natl. Acad. Sci. USA, 87, 6378 (1990); PCT Patent Application Number 96/40189] and peptides on plasmids [United States Patent No. 5,270,170; United States Patent No. 5,338,665].

### [5] Uses of the DNAs and Polypeptides of the Invention

(1) The DNA of the present invention may be used as a probe in Northern hybridization on RNA that is extracted from human tissues or human-derived cells in the same manner as described in (2) of Section [1] above, to thereby detect or quantitatively determine the mRNA of the polypeptide gene of the present invention in the tissues or cells. By comparing the amounts of RNA expressed in various tissues, the tissue distribution of the polypeptide of the present invention can be elucidated.
   Alternatively, the oligonucleotide of the present invention may be used as a primer specific to the DNA of the present invention in RT-PCR [reverse transcription PCR; PCR protocols (1990)] on RNA that is extracted from human tissues or human-derived cells in the same manner as described in (2) of Section [1] above, to thereby detect or quantitatively determine the mRNA of the polypeptide gene of the present invention. These methods of quantitative determination of the mRNA of the polypeptide gene may be used in the diagnosis of disease states in which the gene is involved.
   By quantification of the mRNA encoding the polypeptide in various disease model animals, it is possible to reveal the importance of the gene product in diseases. Furthermore, it is possible to evaluate a drug by comparing the amount of expression of the mRNA encoding the polypeptide in the presence or absence of the drug.
(2) The DNA of the present invention or an oligonucleotide having a nucleotide sequence identical with or complementary to a partial nucleotide sequence of the DNA may be used as a probe to carry out *in situ* hybridization [Methods in Enzymology, 254, 419 (1995)] on human tissue section. As a result, more detailed information on the distribution of the polypeptide of the present invention can be obtained, e.g. cells expressing the polypeptide in a given tissue can be specified.
   Information as to in which tissue or cell the polypeptide of the present invention is expressed, and information as to what stimulation given to cells changes the amount of expression of the polypeptide obtained by the above-described methods will be useful in elucidating the physiological functions of the polypeptide of the present invention and its involvement in diseases.
(3) The DNA of the present invention may be used as a probe to carry out Southern hybridization (Molecular Cloning 2nd Ed.) on genomic DNA. As a result, mutations in the gene encoding the polypeptide of the present invention can be detected. By detecting such mutations, it is possible to diagnose those diseases which may be caused by mutations of the gene. Specifically, with respect to MT4-MMP(2), diseases such as arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, and inflammations associated with infiltration of leukocytes may be diagnosed. With respect to MT5-MMP, diseases such as arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, brain disorders at the time of cerebral apoplexy, Alzheimer's disease, dementia, multiple sclerosis, Parkinson's disease, brain tumor, and inflammations associated with infiltration of leukocytes may be diagnosed.
(4) The anti-sense oligonucleotides (RNA/DNA) of the present invention are expected to be applicable to treatment or prevention of diseases in which the gene encoding the polypeptide of the present invention may be involved in their onset, by inhibiting the transcription of the gene or the translation of the mRNA [Chemistry 46, 681 (1991); Bio Technology, 9, 358 (1992)]. With respect to MT4-MMP(2), specific examples of such diseases include arthrosis deformans, rheumatoid arthritis, asthma, autoimmune diseases, atopic dermatitis, psoriasis, contact dermatitis, alopecia, ischemic heart diseases, organ transplantation, hepatitis, nephritis, pancreatitis, arteriosclerosis, leukemia, malignant tumor, and inflammations associated with infiltration of leukocytes. With respect to MT5-MMP, specific examples of such diseases include arthrosis deformans, rheumatoid arthritis, asthma, autoimmune diseases, atopic dermatitis, psoriasis, contact dermatitis, alopecia, ischemic heart diseases, organ transplantation, hepatitis, nephritis, pancreatitis, arteriosclerosis, leukemia, malignant tumor, brain disorders at the time of cerebral apoplexy, Alzheimer's disease, dementia, multiple sclerosis, Parkinson's disease, brain tumor, and inflammations associated with infiltration of leukocytes.
   The above-described anti-sense oligonucleotide is designed and prepared based on a partial nucleotide sequence of the DNA encoding the polypeptide of the present invention, preferably a nucleotide sequence complementary to 10-50 bases within the translation initiation region, and then administered into the living bodies of subjects.
   Pharmaceuticals containing the DNA of the present invention are prepared or administered in the same manner as described below except that the DNA of the present invention is used instead of the polypeptide of the present invention.
(5) The polypeptide of the present invention can be obtained by using the DNA of the present invention in accordance with the method described in [2] above. With respect to MT4-MMP(2), a polypeptide of the present invention is used for a diagnostic agent, therapeutic agent or prophylactic agent for diseases such as arthrosis deformans, rheumatoid arthritis, asthma, autoimmune diseases, atopic dermatitis, psoriasis, contact dermatitis, alopecia, ischemic heart diseases, organ transplantation, hepatitis, nephritis, pancreatitis, arteriosclerosis, leukemia, malignant tumor, and inflammations associated with infiltration of leukocytes. With respect to MT5-MMP, a polypeptide of the present invention is used for a diagnostic agent, therapeutic agent or prophylactic agent for diseases such as arthrosis deformans, rheumatoid arthritis, asthma, autoimmune diseases, atopic dermatitis, psoriasis, contact dermatitis, alopecia, ischemic heart diseases, organ transplantation, hepatitis, nephritis, pancreatitis, arteriosclerosis, leukemia, malignant tumor, brain disorders at the time of cerebral apoplexy, Alzheimer's disease, dementia, multiple sclerosis, Parkinson's disease, brain tumor, and inflammations associated with infiltration of leukocytes is contemplated.
   Although it is possible to administer the polypeptide of the present invention alone as a diagnostic agent or therapeutic agent, usually it is preferred that the polypeptide of the present invention be administered as a pharmaceutical preparation obtained by mixing the polypeptide with one or more pharmacologically acceptable carriers and formulating by any of the well-known methods in the technical field of pharmaceutics. Preferably, a sterile solution of the polypeptide dissolved in water or an aqueous carrier such as an aqueous solution of NaCl, glycine, glucose or human albumin is used. Pharmacologically acceptable additives such as buffers and isotonic agents that bring liquid preparations close to physiological conditions may also be added. For example, sodium acetate, sodium chloride, sodium lactate, potassium chloride, or sodium citrate may be added. The pharmaceutical preparation may be freeze-dried for storage and dissolved in an appropriate solvent at the time of use.
   It is desirable to select the best route of administration that would be most effective for the treatment intended. Usually, parenteral routes such as subcutaneous, intramuscular, intravenous, or intratracheal route are used.
(6) The DNAs of the present invention (sense DNAs and anti-sense DNAs) or oligonucleotides comprising a part of the nucleotide sequences thereof may be incorporated as a single-stranded or double-stranded DNA or oligonucleotide into viral vectors such as retrovirus, adenovirus, adeno-associated virus or other vectors to prepare vectors for gene therapy, and may be used in such therapy.

### EXAMPLES

Hereinbelow, the present invention will be described more specifically with reference to the following Examples. However, the scope of the present invention is not limited by these Examples.

### EXAMPLE 1. Cloning of the Gene of Mouse MT4-MMP-Related Protein [MT4-MMP(2)]

Since MT4-MMP gene is highly expressed in the human brain, a brain cDNA library from mouse 17-day embryo was prepared using ZAP-cDNA Synthesis Kit (Stratagene) according to the manual attached to the Kit.

Using a partial sequence of the human MT4-MMP gene (positions 233-1899 of SEQ ID NO: 17) as a probe, the resultant cDNA library was screened by plaque hybridization.

Several of the positive clones that hybridized to the above probe were analyzed for their nucleotide sequences. All of the analyzed clones contained a signal peptide sequence that is considered missing in the reported human MT4-MMP gene; the longest clone was 3.5 kb. Therefore, it was considered that an mRNA corresponding to the DNA of SEQ ID NO: 3 which can express the 587 amino acid MT4-MMP(2) shown in SEQ ID NO: 1 is expressed in mouse.

### EXAMPLE 2. Cloning of Human MT4-MMP (2) Gene

EST clones relating to the human MT4-MMP gene were searched for through databases. However, no clones were registered which contain a sequence encoding a signal peptide as seen in the above-mentioned mouse gene. Therefore, it was considered that secretion-type human MT4-MMP gene does not exist or there are reasons that make the isolation thereof difficult.

A human brain cDNA library (Clontech) was screened using a partial sequence of the mouse MT4-MMP(2) gene, as a probe, from Example 1 encoding an N-terminal region representing the signal peptide. However, the gene of interest could not be isolated. Then, the inventors analyzed 5' regions of transcripts by 5' RACE. For this analysis, monocyte-derived THP-1 (ATCC TIB-202; American Type Culture Collection) cells were used in which expression of MT4-MMP mRNA had been confirmed.

Briefly, a cDNA was prepared using poly(A)⁺ RNA isolated from human THP-1 cells, a human MT4-MMP selective primer (SEQ ID NO: 9) and Superscript II (Gibco BRL). A single-stranded oligonucleotide adaptor (SEQ ID NO: 10) was ligated to the resultant cDNA with T4 RNA ligase. Then, a PCR was performed in GC buffer using the MT4-MMP selective primer (SEQ ID NO: 9), an adaptor selective primer (SEQ ID NO: 11) and LA Taq (Takara). After completion of this reaction, another PCR was performed using a gene-selective, other primer (SEQ ID NO: 12) and an adaptor selective primer (SEQ ID NO: 13).

The analysis of the 50 clones revealed that, while 3 clones were cDNA fragments containing an MT4-MMP sequence, 47 clones were cDNA fragments encoding a signal peptide sequence similar to that in mouse MT4-MMP(2). From this, in addition to the downstream region of the propeptide sequence already known, the entire region of the mRNA of SEQ ID NO: 4 encoding human MT4-MMP(2) as shown in SEQ ID NO: 2 containing a signal peptide has been elucidated. Although the nucleotide sequence of an EST clone H97792 was almost identical with the sequence of the MT4-MMP gene reported by Puente [Cancer Research, 56, 944 (1996); SEQ ID NO: 17], a partial sequence of the catalytic domain was different. The EST clone H97792 was more highly conserved with mouse MT4-MMP(2) gene. When the entire sequence of human MT4-MMP(2) gene was determined newly, differences were found even in the previously sequenced region of the MT4-MMP gene reported by Puente.

Mouse and human MT4-MMP(2) genes are mutually conserved well; their propeptide domains, catalytic domains, hinge domains and hemopexin-like domains had 87%, 87%, 78% and 96% homology, respectively. Their signal peptide domains and transmembrane domains had relatively low similarities of 54% and 35%, respectively. When the catalytic domain of human MT4-MMP(2) gene were compared with the catalytic domains of MT1-MMP, MT2-MMP and MT3-MMP, the similarities were 36%, 39% and 31%, respectively. These results also supported that mouse MT4-MMP(2) gene is most close to human MT4-MMP(2) gene. Thus, it was concluded that mouse MT4-MMP(2) gene is a mouse homologue to human MT4-MMP(2) gene.

### EXAMPLE 3. Expression of MT4-MMP(2) and Detection of the Gene Product

In order to confirm that a gene product is certainly translated from the isolated cDNA, the cDNA was integrated into pSG5 vector (Stratagene) containing an SV 40 promoter. For detecting the expressed product, a FLAG sequence (Eastman Chemical) was integrated downstream of the latent enzyme processing site to thereby enable detection with anti-FLAG antibodies.

COS-1 cells were transfected with mouse or human MT4-MMP(2) expression plasmids. After 48 hr, cells were harvested and lysed followed by detection of FLAG-labeled MT4-MMP by Western blotting. With the use of an anti-FLAG antibody M2 (Eastman Chemical), a specific 66 kDa band in both cells transfected with the expression plasmids was detected.

### EXAMPLE 4. Detection and Analysis of MT4-MMP Transcript

Since MT4-MMP transcript has an *Alu* sequence at 5' end, there was a possibility that it contains intron(s). Using a partial sequence of human MT4-MMP(2) (positions 212-519 of SEQ ID NO: 4) as a probe, hybridized clones were isolated from a library of Health Science Research Resources Bank (Deposit No. LI020) by hybridization, and plasmids were extracted from the resultant clones by conventional methods. Then, the present inventors examined nucleotide sequences around the 5' end region (positions 140-272 of SEQ ID: 17) of MT4-MMP contained in these plasmids.

When MT4-MMP gene was compared with MT4-MMP(2) gene, MT4-MMP nucleotide sequence of the region in which homology no longer exists (positions 1-139 of SEQ ID NO: 17) was almost identical with positions 3008-3147 of the genomic sequence (SEQ ID NO: 18); and a splice donor sequence was found on the border between the region with homology and the region without homology. The sequence encoding the exons of MT4-MMP (positions 140-340 of SEQ ID NO: 17) were almost identical with positions 3148-3280 and positions 3564-3633 of the genomic sequence (SEQ ID NO: 18). From these results, it was concluded that the transcript still containing the first intron is MT4-MMP transcript.

From these results, it was considered that two mRNAs encoding MT4-MMP and MT4-MMP(2) are expressed in human.

In order to discriminate these two transcripts by performing RT-PCR separately, 5' primers specific to individual transcripts (MT4-MMP: SEQ ID NO: 14; MT4-MMP(2): SEQ ID NO: 15) and a common 3' primer (SEQ ID NO: 16) were prepared.

The expression of these transcripts in various cancer cells is shown in Table 1 below.

**Table 1.**

| Expression of MT4-MMP(2) and MT4-MMP Transcripts in Cancer Cells | | | |
|---|---|---|---|
| Cancer Cell Line | MT4-MMP(2) | MT4-MMP | Accession Number |
| Jurkat (T cell) | ++ | +/- | ATCC TIB-152 |
| Raji (B cell) | - | - | ATCC CCL-86 |
| BJAB (B cell) | - | - | ATCC HB-136 |
| THP-1 (monocytic) | ++ | + | ATCC TIB-202 |
| K562 (monocytic) | ++ | - | ATCC CCL-243 |
| U-937 (monocytic) | ++ | - | ATCC CRL-1593.2 |
| U-251 MG (astrocytoma) | ++ | - | Hakkoken IFO50288 |
| SK-N-SH (neuroblastoma) | ++ | - | ATCC HTB-11 |
| no.10 (glioma) | +/- | - | Hakkoken IFO50368 |
| KALS-1 (glioma) | ++ | - | Hakkoken IFO50434 |
| MKN-7 (gastric) | + | - | Riken RCB0999 |
| MKN-28 (gastric) | - | - | Riken RCB1000 |
| NUGC-4 (gastric) | + | - | HS Found JCRB0834 |
| PANC-1 (pancreatic) | ++ | + | ATCC CRL-1469 |
| MIA PaCa-2 (pancreatic) | ++ | +/- | ATCC CRL-1420 |
| SK-HEP-1 (hepatoma) | ++ | + | ATCC HTB-52 |
| Hep 3B (hepatoma) | ++ | + | ATCC HB-8064 |
| ZR-75-1 (breast) | ++ | + | ATCC CRL-1500 |
| MCF7(adenocarcinoma) | ++ | + | ATCC HTB-22 |
| T-24 (bladder) | ++ | + | ATCC HTB-4 |
| A375 (melanoma) | ++ | + | ATCC CRL-1619 |
| HT-1080 (fibrosarcoma) | + | - | ATCC CCL-121 |
| ++: strong expression; +: medium expression; +/-: slight expression; -: no expression | | | |
| ATCC: American Type Culture Collection | | | |
| HS Found.: Japan Health Sciences Foundation | | | |
| Riken: The Institute of Physical and Chemical Research | | | |
| Hakkoken: Institute for Fermentation, Osaka | | | |

MT4-MMP was only expressed in those cells where expression of MT4-MMP(2) was recognized.

From these results, it is believed that MT4-MMP(2) is the major transcript and that expression of MT4-MMP also occurs depending on cells under similar transcriptional control.

### EXAMPLE 5. Expression of MT4-MMP(2) in mouse Tissues

Tissues of 4-week old mice were excised by organ. RNA was extracted therefrom and used to examine the expression pattern of MT4-MMP(2). Briefly, 20 *µ*g of total RNA was electrophoresed on 1% agarose gel and transferred onto a nylon membrane followed by Northern blot analysis using ³²P-labeled mouse MT4-MMP(2) gene as a probe, to thereby examine the expression pattern of MT4-MMP(2).

Organs in which particularly high expression was observed were the cerebrum, cerebellum, brainstem, large intestine, uterus, and testis. Little expression was observed in the adrenal, mammary gland, and placenta. The results of expression in mouse were consistent with the results of MT4-MMP expression in human tissues reported by Puente et al. [Cancer Research, 56, 944 (1996)].

In mouse, the expression of MT4-MMP(2) was very high in the brain, and its expression was also observed in some limited organs such as the large intestine, uterus and testis. This presents a contrast to the expression of MT1-MMP and MT2-MMP seen in a relatively wide range of tissues. From this, it is believed that MT4-MMP(2) is involved in the maintenance of homeostasis in tissues through the degradation of extracellular substrates specific to those organs expressing MT4-MMP(2).

### EXAMPLE 6. Expression of a Mouse MT4-MMP(2) Partial Peptide (Hemopexin-like Domain) in E. coli

A cDNA encoding a mouse MT4-MMP(2) partial peptide (hemopexin-like domain) having an amino acid sequence represented by positions 321-550 of SEQ ID NO: 1 to which a methionine residue was added at the N-terminus was amplified by polymerase chain reaction (PCR) using the cDNA of mouse MT4-MMP(2) as a template.

The amplified fragment was subcloned into an *E. coli* expression vector pET3a (Takara) and then introduced into *E. coli* BL21 (DE3) pLysS (Takara). This *E. coli* was grown in 1 liter of expression medium in the presence of 100 *µ* g/ml of ampicillin until OD₆₀₀ reached 0.5. Then, the cells were stimulated with 0.4 mmol/L of isopropyl- β -D-thiogalactopyranoside (IPTG) and cultured for another three hours.

After the culturing, granules (inclusion bodies) consisting of the mouse MT4-MMP(2) partial peptide formed in *E. coli* cells were collected by conventional methods and dissolved in a solubilization solution containing 8 mol/L urea, 50 mmol/L Tris-HCl (pH 8.6) and 20 mmol/L dithiothreitol (DTT). The resultant solution was applied to High Q anion exchange column followed by recovery of the fraction eluted with 0.2 mol/L sodium chloride.

This fraction was diluted with a solution containing 50 mmol/L Tris-HCl (pH 8.6), 6 mol/L urea, 1 mmol/L dithiothreitol, 0.15 mol/L sodium chloride, 5 mmol/L calcium chloride, 100 mmol/L zinc chloride and 0.02% sodium azide. Then, cystamine (final concentration: 20 mmol/L) was added to the resultant dilution. Subsequently, the resultant solution was dialyzed against a solution containing 50 mmol/L Tris-HCl (pH 8.6), 0.15 mol/L sodium chloride, 5 mmol/L calcium chloride, 100 mmol/L zinc chloride, 5 mmol/L *β* mercaptoethanol, 1 mmol/L 2-hydroxyethyldisulfide and 0.02% sodium azide at 4°C. Further, dialysis was performed against 10 volumes of a solution containing 50 mmol/L Tris-HCl (pH 7.5), 0.15 mol/L sodium chloride, 5 mmol/L calcium chloride, 50 mmol/L zinc chloride and 0.02% sodium azide (4 hr x 3 times). The dialyzed solution was centrifuged at 22,000xg at 4°C for 10 min to remove the precipitate.

The supernatant was applied to S-200 column pre-equilibrated with a buffer containing 50 mmol/L Tris-HCl (pH 7.5), 150 mmol/L sodium chloride, 10 mmol/L calcium chloride and 0.02% sodium azide for gel filtration to obtain a mouse MT4-MMP(2) partial peptide corresponding to the hemopexin-like domain.

### EXAMPLE 7. Expression of a Human MT4-MMP(2) Partial Peptide (Propeptide Domain + Active Domain) in E. coli

A cDNA encoding a human MT4-MMP(2) partial peptide having an amino acid sequence represented by positions 58-298 of SEQ ID NO: 2 was amplified by polymerase chain reaction (PCR) using the cDNA of human MT4-MMP(2) as a template.

The amplified fragment was subcloned into an E. *coli* expression vector pRSET (Invitrogen). The enzyme was expressed as a fusion protein fused to the 6x His sequence present in the pRSET-derived leader sequence. This vector was introduced into *E. coli* BL21 (DE3) pLysS (Takara). This E. *coli* was grown in 1 liter of expression medium (tryptone, 12 g/L; yeast extract, 24 g/L; sodium chloride, 10 g/L; Trisma base, 250 mg/L; glycerol, 4 ml/L) in the presence of 100 *µ*g/ml ampicillin until OD₆₀₀ reached 0.5. Then, the cells were stimulated with 0.4 mmol/L isopropyl-*β*-D-thiogalactopyranoside (IPTG) and cultured for another three hours.

After the culturing, granules (inclusion bodies) consisting of the human MT4-MMP(2) partial peptide (propeptide domain + active domain) formed in *E. coli* cells were collected by conventional methods and dissolved in a solubilization solution containing 8 mol/L urea and 50 mmol/L Tris-HCI (pH 8.6). The resultant solution was applied to a nickel chelate column followed by recovery of the fraction eluted with 250 mmol/L imidazole.

This fraction was diluted with a solution containing 50 mmol/L Tris-HCl (pH 8.6), 6 mol/L urea, 20 mmol/L dithiothreitol, 0.15 mol/L sodium chloride, 100 mmol/L calcium chloride, 100 *µ*mol/L zinc chloride and 0.02% sodium azide (200-fold dilution). Then, cystamine (final concentration: 20 mmol/L) was added to the resultant dilution, Subsequently, the resultant solution was dialyzed against a solution containing 50 mmol/L Tris-HCl (pH 8.6), 0.15 mol/L sodium chloride, 10 mmol/L calcium chloride, 100 *µ* mol/L zinc chloride, 5 mmol/L β mercaptoethanol, 1 mmol/L 2-hydroxyethyldisulfide and 0.02% sodium azide at 4°C. Further, dialysis was performed against 10 volumes of a solution containing 50 mmol/L Tris-HCl (pH 7.5), 0.15 mol/L sodium chloride, 10 mmol/L calcium chloride, 50 *µ* mol/L zinc chloride and 0.02% sodium azide (4 hr x 3 times). The thus dialyzed solution was centrifuged at 22,000xg at 4°C for 10 min to remove the precipitate.

The supernatant obtained was concentrated 5-fold with Amicon YM-10 (Millipore) to prepare a crude enzyme.

### EXAMPLE 8. Measurement of the Activity of the Human MT4-MMP(2) Partial Peptide (Active Domain)

### a) Activation of the Human MT4-MMP(2) Partial Peptide (Propeptide Domain + Active Domain)

It is known that MMPs are activated by trypsin treatment and then exhibit metalloproteinase activity. Whether MT4-MMP(2) is also activated by such treatment or not was examined by the method described below.

Briefly, trypsin (Wako Purechemical Industries, Ltd.) was added to 200 *µ*l of the crude MT4-MMP(2) partial peptide (propeptide domain + active domain) solution to give a concentration of 0.1 *µ*g/ml, and reacted at 37°C for 30 min. Then, phenylmethanesulfonyl fluoride (PMSF) (a serine protease inhibitor) was added to the reaction solution at 1 mmol/L to inactivate the trypsin.

### b) Assay

To 10 *µ*l of the activated enzyme, a measurement buffer or an inhibitor diluted with a measurement buffer (TIMP-1 or TIMP-2; final concentration: 1 *µ*g/ml) was added to make a 50 *µ*l solution. To this solution, 50 *µ*l of 10 *µ*mol/L fluorescent substrate was iadded and reacted at 37°C for 120 min. After the completion of each reaction, fluorescence generated by the enzyme reaction was measured. Measurement was carried out under the following conditions: excitation wave length: 320 nm; fluorescence wave length: 395 nm.

The reagents and substrates used in this assay were as described below.
- Fluorescent substrate:: DMSO stock (10 mmol/L); MOCAc-Pro-Leu-Gly-Leu-A₂pr(Dnp)-Ala-Arg-NH₂ (Peptide Institute, Inc.)
- Standard fluorescent substrate:: DMSO stock (1 mmol/L); MOCAc-Pro-Leu-Gly (Peptide Institute, Inc.)
- Activity measurement buffer:: 0.1 mol/L Tris-HCl (pH 7.5; Nacalai Tesque), 0.1 mol/L NaCl (Nacalai Tesque), 0.01 mol/L CaCl₂ (Wako Purechemical), 0.05% Briji-35 (w/v; Wako Purechemical)

The results of the measurement are shown in Table 2. Similar to other MMPs, the MT4-MMP(2) partial peptide, in particular, the partial peptide after the activation by trypsin exhibited a strong substrate-degrading activity.

It is reported that MT-MMPs are not inhibited by the metalloproteinase inhibitor TIMP-1, but inhibited by TIMP-2 [FEBS Letters, 393, 101 (1996)].

Whether MT4-MMP(2) also has such a nature or not was examined.

As shown in Table 2, similar to the activities of other MT-MMPs, the activity of MT4-MMP(2) was not inhibited by TIMP-1, but strongly inhibited by TIMP-2.

These results revealed that MT4-MMP(2) is one type of MT-MMP.

**Table 2.**

| Measurement of the Activity of Human MT4-MMP(2) Partial Peptide (Active Domain) | | | |
|---|---|---|---|
| Sample | Trypsin Treatment | Inhibitor | Fluorescence Intensity (Mean ± SD) (n=3) |
| Blank | | | 0.000*±*0.057 |
| Human MT4-MMP(2) partial peptide | - | None | 1.304±0.056 |
| Human MT4-MMP(2) partial peptide | + | None | 4.882±0.102 |
| Human MT4-MMP(2) partial peptide | + | TIMP-1 | 3.493±0.166 |
| Human MT4-MMP(2) partial peptide | + | TIMP-2 | 0.076±0.065 |

### EXAMPLE 9. Cloning of Mouse MT5-MMP Gene

In order to isolate mouse MT5-MMP gene, a brain cDNA library from mouse 17-day embryo was prepared using ZAP-cDNA Synthesis Kit (Stratagene) according to the manual attached to the kit.

The resultant cDNA library was screened by plaque hybridization using human MT3-MMP gene as a probe. Clones exhibiting a strong signal and clones exhibiting a weak signal were obtained. The nucleotide sequences of these clones were determined.

As a result of analysis of clones with a weak signal, a 2.1 kb sequence was found in one of them. Although this sequence exhibited weak homology to human and rat MT3-MMP genes, it was not homologous to other MMP genes. Thus, it was considered that this sequence represents a novel MMP gene.

Subsequently, a 3.7 kb cDNA fragment that hybridized to the above-described 2.1 kb sequence was obtained from the above library by plaque hybridization. From the 2.1 kb and 3.7 kb sequences, a 4.2 kb cDNA sequence shown in SEQ ID NO: 7 was obtained.

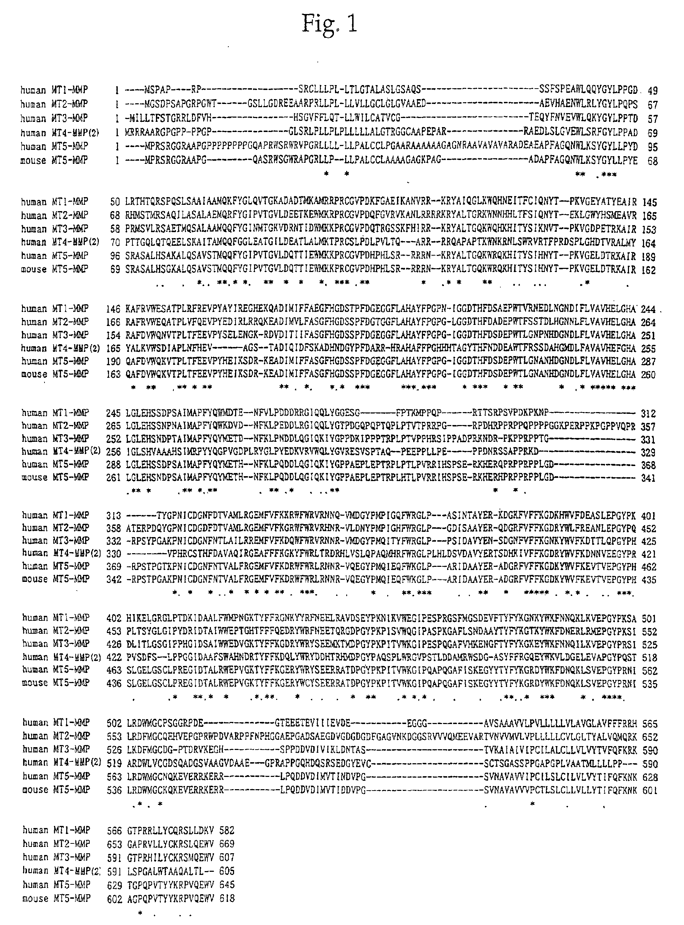

A protein with 618 amino acids represented by SEQ ID NO: 5 was encoded in the cDNA shown in SEQ ID NO: 7. Since the peptide of SEQ ID NO: 5 contains those sequences corresponding to the individual domains of MT-MMPs in well-conserved states, it was concluded that this peptide is a novel MT-MMP, namely, mouse MT5-MMP (Fig. 1).

### EXAMPLE 10. Cloning of Human MT5-MMP Gene

In order to confirm the human gene corresponding to mouse MT5-MMP gene, a human kidney cDNA library (Clontech) was screened by plaque hybridization using mouse MT5-MMP gene as a probe in the same manner as in Example 9. As a result, a gene that has 92% homology to mouse MT5-MMP gene and is different from known MT-MMP genes was obtained.

All of the sequenced human MT5-MMP cDNA clones lacked a 5' region that is supposed to encode a signal peptide. Thus, the sequence of the missing region was determined by 5' RACE as described below to thereby determine the nucleotide sequence containing the entire region encoding human MT5-MMP gene.

Briefly, cDNA was prepared from a human brain poly(A)⁺ RNA (Clontech) using Superscript II (Gibco BRL) and a human MT5-MMP gene-selective primer (SEQ ID NO: 19) according to the manual attached to the kit.

A single-stranded oligonucleotide adaptor (SEQ ID NO: 10) was ligated to the resultant cDNA with T4 RNA ligase. Then, the cDNA was subjected to PCR in GC buffer using the MT5-MMP gene-selective primer (SEQ ID NO: 19), an adaptor-selective primer (SEQ ID NO: 11) and LA Taq (Takara).

After completion of the above PCR, another PCR was performed using an other gene-selective primer (SEQ ID NO: 20) and an adaptor-selective primer (SEQ ID NO: 13). From the above-mentioned sequence obtained from the human kidney cDNA library using the mouse gene as a probe and the sequence obtained from the 5' RACE, a 2.6 kb cDNA fragment (shown in SEQ ID NO: 8) that encodes a 645 amino acid protein (shown in SEQ ID NO: 6) was obtained.

### EXAMPLE 11. Expression of MT5-MMP mRNAs in Internal Organs

Expression of MT5-MMP gene in tissues was examined by Northern blotting.

Briefly, 20 *µ*g of total RNA was electrophoresed on 1 % agarose gel and transferred onto a nylon membrane. Then, Northern blotting was carried out using ³²P-labeled mouse MT5-MMP gene as a probe to examine the expression pattern of approximately 4 kb MT5-MMP mRNAs.

In 2-week old mice, a strong expression was observed only in the brain, but the expression was around detection limit or below in other tissues of other organs.

When expression in human tissues was examined with Multiple Tissue Blot (Clontech) using human MT5-MMP gene as a probe, high expression was observed in the brain. The results of Northern blotting using ³²P-labeled human MT5-MMP gene as a probe revealed that strong expression of both 4.0 kb and 4.8 kb MT5-MMP mRNAs are also recognized in the human brain. In human, the expression was also recognized in the kidney and pancreas. The 4.8 kb mRNA and the 4.0 kb mRNA were expressed strongly in the brain and in the kidney and pancreas, respectively.

Then, RT-PCR was carried out using MT5-MMP specific primers (SEQ ID NOS: 21 and 22) to analyze these fragments. As a result, it was found that a DNA fragment of the same size as that of the fragment amplified in the brain is amplified in the kidney and pancreas with almost equal efficiencies and that no products of different sizes were found. Thus, it was believed that the shorter transcript contains the entire coding region.

When the expression of MT5-MMP in mouse and human was examined, characteristic expression was observed in the brain. In particular, the expression was limited in the brain in mouse, and was very low in other internal organs.

Since the expression of this gene in the brain is characteristic, site-specific expression was examined using Human Brain Multiple Tissue Blot (Clontech).

High expression of MT5-MMP was observed in the cerebellum. Its expression was also observed in the cerebral cortex, medulla, occipital region of head, frontal region of head, temporal region of head and putamen, but not observed in the spinal cord.

These results show a remarkable characteristic of MT5-MMP gene different from other MT-MMP genes expressed in various tissues.

In human, the expression of MT5-MMP was strong in the brain, and its expression was observed in the kidney and pancreas. The results of examination of its site-specific expression in the human brain revealed a characteristic expression in the cerebellum, High expression in the cerebellum was also confirmed in mouse.

These results suggest the possibility that MT5-MMP controls the degradation of extracellular matrixes around cells associated with such processes as the maturation and maintenance of brain tissues, the construction of nervous network, and so forth.

### EXAMPLE 12, Expression of MT5-MMP mRNA in Cancer Cells

MT1-MMP is expressed frequently in cancer cells *per* se and interstitial cells around them in many cancer tissues and functions as an activator of gelatinase A at the tissue level. The expression of MT5-MMP in various cancer cell strains was examined by RT-PCR using MTS-MMP-specific primers (SEQ ID NOS: 21 and 22).

The results are shown in Table 3 below.

**Table 3.**

| Expression of MT5-MMP Transcript in Cancer Cells | | |
|---|---|---|
| Cancer Cell Line | MT5-MMP | Accession Number |
| Jurkat (T cell) | - | ATCC TIB-152 |
| Raji (B cell) | - | ATCC CCL-86 |
| BJAB (B cell) | - | ATCC HB-136 |
| THP-1 (monocytic) | - | ATCC TIB-202 |
| K562 (monocytic) | - | ATCC CCL-243 |
| U-937 (monocytic) | - | ATCC CRL-1593.2 |
| U-251 MG (astrocytoma) | - | Hakkoken IFO50288 |
| SK-N-SH (neuroblastoma) | +++ | ATCC HTB-11 |
| no.10 (glioma) | ++ | Hakkoken IFO50368 |
| KALS-1 (glioma) | +++ | Hakkoken IFO50434 |
| MKN-7 (gastric) | + | Riken RCB0999 |
| MKN-28 (gastric) | - | Riken RCB1000 |
| NUGC-4 (gastric) | + | HS Found 3CRB0834 |
| PANC-1 (pancreatic) | + | ATCC CRL-1469 |
| MIA PaCa-2 (pancreatic) | + | ATCC CRL-1420 |
| SK-HEP-1 (hepatoma) | + | ATCC HTB-52 |
| Hep 3B (hepatoma) | + | ATCC HB-8064 |
| ZR-75-1 (breast) | ? | ATCC CRL-1500 |
| MCF7(adenocarcinoma) | - | ATCC HTB-22 |
| T-24 (bladder) | - | ATCC HTB-4 |
| A375 (melanoma) | +/- | ATCC CRL-1619 |
| HT-1080 (fibrosarcoma) | +/- | ATCC CCL-121 |
| +++: very strong expression; ++: strong expression; +: medium expression; +/-: slight expression; -: no expression | | |
| ATCC: American Type Culture Collection | | |
| HS Found.: Japan Health Sciences Foundation | | |
| Riken: The Institute of Physical and Chemical Research | | |
| Hakkoken: Institute for Fermentation, Osaka | | |

While MT1-MMP is expressed in various cancer cell lines, cell lines expressing MT5-MMP were specific in the nervous system-derived neuroblastoma [SK-N-SH (HTB-11, ATCC)], undifferentiated glioma [no. 10 (IFO50368, Institute for Fermentation, Osaka)], and glioma [KALS-1, (IFO50434, Institute for Fermentation, Osaka)], with the correlation of the expression in brain.

Also, its expression in pancreatic cancer cell strains [PANC-1 (CRL-1469, ATCC); MIA PaCa-2 (CRL-1420, ATCC)] and hepatoma cell strains [SK-HEP-1 (HTB-52, ATCC): Hep 3B (HB-8064, ATCC)] was characteristic.

It is considered that abnormal expression of MT-MMPs on cell surfaces promotes the infiltration of cells. Actually, excessive expression of MT1-MMP enhances the infiltrating ability of cancer cell lines and increases the frequency of experimental metastasis. In human cancer tissues, cancer cells and fibroblasts around them express MT1-MMP at high frequency, and the presence of gelatinase A which MT1-MMP activates at sites of its expression is well correlated with the infiltration and metastasis of cancer.

Since MT5-MMP is expressed in undifferentiated glioma, glioma, pancreatic cancer and hepatoma cell lines, the possibility has been suggested that excessive expression of MT5-MMP is involved in the malignant nature of cancer cells in a specific types of cancers.

### EXAMPLE 13. Expression of a Mouse MT5-MMP Partial Peptide (Propeptide Domain + Active Domain) in E. coli

A cDNA encoding a mouse MT5-MMP partial peptide having an amino acid sequence represented by positions 40-300 of SEQ ID NO: 5 to which a methionine residue is added at the N terminus was amplified by polymerase chain reaction (PCR) using the cDNA of mouse MT5-MMP as a template.

The amplified fragment was subcloned into an *E. coli* expression vector pET3a (Takara) and then introduced into *E. coli* BL21 (DE3) pLysS (Takara). This *E. coli* was grown in 1 liter of expression medium (12 g/L of tryptone; 24 g/L of yeast extract; 10 g/L of sodium chloride; 250 mg/L of Trisma base; 4 ml/L of glycerol) in the presence of 100 *µ*g/mL ampicillin until OD₆₀₀ reached 0.5. Then, the cells were stimulated with 0.4 mmol/L isopropyl-β-D-thiogalactopyranoside (IPTG) and cultured for another three hours.

After the culturing, granules (inclusion bodies) consisting of the mouse MT5- MMP partial peptide formed in the *E. coli* cells were recovered by conventional methods and dissolved in a solubilization solution containing 8 mol/L urea, 50 mmol/L Tris-HCl (pH 8.6) and 20 mmol/L dithiothreitol (DTT). The resultant solution was applied to Q-anion ion exchange column followed by recovery of the fraction eluted with 0.1 M NaCl.

This fraction was diluted with a solution containing 50 mmol/L Tris-HCl (pH 8.6), 6 mol/L urea, 1 mmol/L dithiothreitol, 0.15 mol/L sodium chloride, 5 mmol/L calcium chloride, 100 mmol/L zinc chloride and 0.02% sodium azide. Then, cystamine (final concentration: 20 mmol/L) was added to the resultant dilution. Subsequently, the resultant solution was dialyzed against 4 L of a solution containing 50 mmol/L Tris-HCl (pH 8.6), 0.15 mol/L sodium chloride, 5 mmol/L calcium chloride, 100 mmol/L zinc chloride, 5 mmol/L *β* mercaptoethanol, 1 mmol/L 2-hydroxyethyldisulfide and 0.02% sodium azide at 4°C overnight, Further, dialysis was performed against 10 volumes of a solution containing 50 mmoUL Tris-HCl (pH 7.5), 0.15 mol/L sodium chloride, 5 mmol/L calcium chloride, 50 *µ* mol/L zinc chloride and 0.02% sodium azide (4 hr x 3 times). The thus dialyzed solution was centrifuged at 22,000xg at 4°C for 10 min to remove the precipitate.

The supernatant obtained was concentrated with Amicon YM-10 (Millipore) and treated with 0.1 *µ*g/ml trypsin at 37°C for 30 min. After inactivation of the trypsin with 1 mmol/L DTT, the sample was applied to S-200 column pre-equilibrated with a buffer containing 50 mmol/L Tris-HCl (pH 7.5), 150 mmol/L sodium chloride, 10 mmol/L calcium chloride and 0.02% sodium azide to perform gel filtration. As a result, the mouse MT5-MMP partial peptide (propeptide domain + active domain) was obtained.

Human MT5-MMP peptide can also be expressed in the same manner.

### EXAMPLE 14. Measurement of the Activity of Mouse MT5-MMP Partial Peptide (Active Domain)

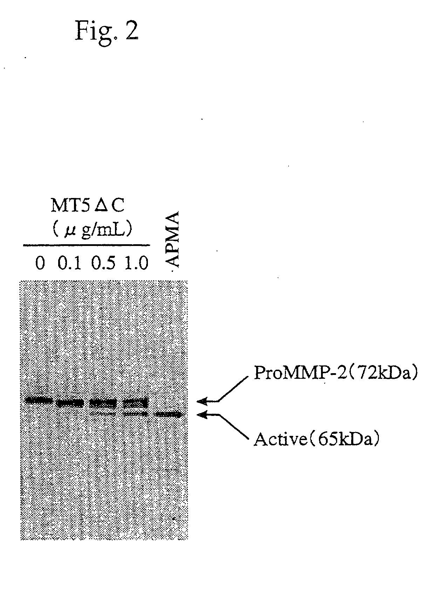

ProMMP-2 (final concentration: 1 *µ*g/mL) and the mouse MT5-MMP partial peptide (propeptide domain + active domain) (final concentration: 1 *µ*g/µL) were mixed and incubated at 37°C for 1 hr. In this operation, Briji 35-added TNC buffer [50 mmol/L Tris-HCl (pH 7.5), 150 mmol/L NaCl, 10 mmol/L CaCl₂, 0.02% NaN₃, 0.05% Briji 35] was used. After the incubation, an equal volume of SDS/PAGE loading buffer was added to the sample, which was then electrophoresed and subjected to Coomassie staining according to routine procedures. As a positive control of activation for ProMMP-2, p-aminophenylmercuric acetate (APMA) was used. As a result, activation of ProMMP-2 was recognized depending on a MMP concentration. The results are shown in Fig. 2.

### INDUSTRIAL APPLICABILITY

By using the DNA of novel MT4-MMP(2) polypeptide obtained by the present invention, it becomes possible to diagnose, prevent or treat diseases such as arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, wounds including corneal ulcer, leukemia, cancer, and inflammations associated with infiltration of leukocytes.

Furthermore, by using the DNA of novel MT5-MMP polypeptide obtained by the present invention, it becomes possible to diagnose, prevent or treat diseases such as arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, wounds including corneal ulcer, leukemia, brain disorders at the time of cerebral apoplexy, Alzheimer's disease, dementia, multiple sclerosis, Parkinson's disease, brain tumor, cancer, and inflammations associated with infiltration of leukocytes.

## Claims

1. A polypeptide consisting of the amino acid sequence as shown in SEQ ID NO: 1.

2. A polypeptide having an amino acid sequence wherein one or several amino acids are deleted, substituted or added in the amino acid sequence of the polypeptide of claim 1 and having metalloproteinase activity.

3. A polypeptide consisting of the amino acid sequence as shown in SEQ ID NO: 2.

4. A polypeptide having an amino acid sequence wherein one or several amino acids are deleted, substituted or added in the amino acid sequence of the polypeptide of claim 3 and having metalloproteinase activity.

5. A polypeptide consisting of the amino acid sequence as shown in SEQ ID NO: 5.

6. A polypeptide having an amino acid sequence wherein one or several amino acids are deleted, substituted or added in the amino acid sequence of the polypeptide of claim 5 and having metalloproteinase activity.

7. A polypeptide consisting of the amino acid sequence as shown in SEQ ID NO: 6.

8. A polypeptide having an amino acid sequence wherein one or several amino acids are deleted, substituted or added in the amino acid sequence of the polypeptide of claim 7 and having metalloproteinase activity.

9. A DNA encoding the polypeptide of any one of claims 1 to 4.

10. A DNA encoding the polypeptide of any one of claims 5 to 8.

11. A DNA consisting of the nucleotide sequence of positions 86-1846 of SEQ ID NO: 3 or positions 100-1917 of SEQ ID NO: 4, or a DNA which hybridizes to said DNA under stringent conditions and which encodes a polypeptide having metalloproteinase activity.

12. A DNA consisting of the nucleotide sequence of positions 75-1928 of SEQ ID NO: 7 or positions 1-1935 of SEQ ID NO: 8, or a DNA which hybridizes to said DNA under stringent conditions and which encodes a polypeptide having metalloproteinase activity.

13. A recombinant DNA that is obtained by integrating the DNA of any one of claims 9 to 12 into a vector.

14. A transformant comprising the recombinant DNA of claim 13.

15. The transformant of claim 14, wherein said transformant is a microorganism belonging to the genus *Escherichia.*

16. The transformant of claim 15, wherein said microorganism belonging to the genus *Escherichia is Escherichia coli.*

17. A method of producing the polypeptide of any one of claims 1 to 8, comprising culturing a transformant comprising a recombinant DNA obtained by integrating a DNA encoding said polypeptide into a vector in a medium, allowing said polypeptide to be produced and accumulated in the resultant culture, and recovering said polypeptide from said culture.

18. An oligonucleotide selected form an oligonucleotide having a nucleotide sequence identical with a nucleotide sequence consisting of consecutive 5 to 60 bases of the DNA of claim 9 or 11; an oligonucleotide having a nucleotide sequence complementary to the nucleotide sequence of said oligonucleotide; or an oligonucleotide derivative of any one of the above oligonucleotides.

19. An oligonucleotide selected form an oligonucleotide having a nucleotide sequence identical with a nucleotide sequence consisting of consecutive 5 to 60 bases of the DNA of claim 10 or 12; an oligonucleotide having a nucleotide sequence complementary to the nucleotide sequence of said oligonucleotide; or an oligonucleotide derivative of any one of the above oligonucleotides.

20. A method of detecting an mRNA encoding the polypeptide of any one of claims 1 to 8 using the oligonucleotide of claim 18 or 19.

21. A method of inhibiting expression of the polypeptide of any one of claims 1 to 8 using the oligonucleotide of claim 18 or 19.

22. A method of screening for an inhibitor or an activator of the polypeptide of any one of claims 1 to 8, which comprises using the polypeptide and a cell that expresses the polypeptide.

23. A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury or inflammations associated with infiltration of leukocytes, wherein said agent comprises the polypeptide of any one of claims 1 to 4.

24. A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury, inflammations associated with infiltration of leukocytes, brain disorders at the time of cerebral apoplexy, Alzheimer's disease, dementia, multiple sclerosis, Parkinson's disease or brain tumor, wherein said agent comprises the polypeptide of any one of claims 5 to 8.

25. A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury or inflammations associated with infiltration of leukocytes, wherein said agent comprises the DNA of claim 9 or 11.

26. A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury, inflammations associated with infiltration of leukocytes, brain disorders at the time of cerebral apoplexy, Alzheimer's disease, dementia, multiple sclerosis, Parkinson's disease or brain tumor, wherein said agent comprises the DNA of claim 10 or 12.

27. A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury or inflammations associated with infiltration of leukocytes, wherein said agent comprises the oligonucleotide of claim 18.

28. A diagnostic agent, therapeutic agent or prophylactic agent for arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury, inflammations associated with infiltration of leukocytes, brain disorders at the time of cerebral apoplexy, Alzheimer's disease, dementia, multiple sclerosis, Parkinson's disease or brain tumor, wherein said agent comprises the oligonucleotide of claim 19.

29. A vector for gene therapy for treating arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury or inflammations associated with infiltration of leukocytes, wherein said vector is obtained by integrating the DNA of claim 9 or 11, or the oligonucleotide of claim 18 into a vector.

30. A vector for gene therapy for treating arthrosis deformans, rheumatoid arthritis, asthma, autoimmune diseases, atopic dermatitis, psoriasis, contact dermatitis, alopecia, ischemic heart diseases, immune response associated with organ transplantation, hepatitis, nephritis, pancreatitis, arteriosclerosis, leukemia, malignant tumor, wounds, corneal ulcer, tissue injury, inflammations associated with infiltration of leukocytes, brain disorders at the time of cerebral apoplexy, Alzheimer's disease, dementia, multiple sclerosis, Parkinson's disease or brain tumor, wherein said vector is obtained by integrating the DNA of claim 10 or 12, or the oligonucleotide of claim 19 into a vector.

31. A method of screening for a compound that regulates the expression of a gene encoding the polypeptide of any one of claims 1 to 8, which comprises contacting a cell that expresses the polypeptide with a test sample.

32. The method of claim 31, wherein said compound that regulates the expression of a gene is detected by determining the amount of mRNA encoding the polypeptide of any one of claims 1 to 8.
